# EUROPEAN PATENT APPLICATION

(11) **EP 4 772 880 A1**
(43) Date of publication of application: **08.07.2026**
(21) Application number: 24859693.4
(22) Date of filing: 26.08.2024
(51) Int. Cl.: G01N 33/53, A61K 35/17, A61P 7/00, A61P 35/00, C12N 5/0783, C12Q 1/06, G01N 33/15, G01N 33/50

(54) **METHOD FOR QUANTIFYING NEOPLASTIC FOLLICULAR REGULATORY T CELLS AND NEOPLASTIC FOLLICULAR CYTOTOXIC T CELLS, METHOD FOR SCREENING NEOPLASTIC FOLLICULAR REGULATORY T CELLS AND NEOPLASTIC FOLLICULAR CYTOTOXIC T CELLS, THERAPEUTIC COMPOSITION, AND KIT**

(30) Priority: 31.08.2023 JP 2023141172
(71) Applicant: University of Tsukuba, Ibaraki 305-8577 (JP)
(72) Inventor: YANAGIMOTO Mamiko, Tsukuba-shi, Ibaraki 305-8577 (JP); ABE Yoshiaki, Tsukuba-shi, Ibaraki 305-8577 (JP)
(74) Representative: Potter Clarkson
(86) International application number: PCT/JP2024/030226
(87) International publication number: WO 2025/047660

(57) **Abstract**

The present invention provides a technique useful for predicting prognosis of patients with follicular lymphoma. The present invention provides a method for quantifying neoplastic follicle regulatory T cells and neoplastic follicle cytotoxic T cells among T cells collected from a subject, for predicting prognosis of follicular lymphoma, the method comprising a flow cytometry step of detecting and enumerating neoplastic follicle regulatory T cells or neoplastic follicle cytotoxic T cells among T cells collected from a subject by a flow cytometry method using an anti-CD4 antibody, an anti-CD8a antibody, an anti-PD-1 antibody, an anti-CD25 antibody, and an anti-TIM-3 antibody, or a multiplex immunostaining step of detecting and enumerating neoplastic follicle regulatory T cells or neoplastic follicle cytotoxic T cells among T cells collected from a subject by a multiplex immunoassay using an anti-CD4 antibody, an anti-CD8 antibody, an anti-PD-1 antibody, an anti-FOXP3 antibody, an anti-TCF-1 antibody, and an anti-Granzyme K antibody.

## Description

### TECHNICAL FIELD

The present invention relates to a method for quantifying neoplastic follicle regulatory T cells and neoplastic follicle cytotoxic T cells among T cells collected from a subject, a method for screening neoplastic follicle regulatory T cells and neoplastic follicle cytotoxic T cells from T cells collected from a subject, a therapeutic composition, a kit, a method for assisting in prognosis prediction of follicular lymphoma, and a method for predicting prognosis of follicular lymphoma in a subject.

This application claims priority based on Japanese Patent Application No. 2023-141172, filed on August 31, 2023, the contents of which are incorporated herein by reference.

### BACKGROUND ART

Follicular lymphoma (FL) is a typical indolent (low-grade) B-cell lymphoma that accounts for 10-20% of all non-Hodgkin's lymphomas. The incidence of FL in Japan has been increasing recently. FL is histopathologically classified as grade 1, 2, 3A, or 3B, with grade 3B usually treated as aggressive (intermediate- to high-grade) lymphoma. Most patients are diagnosed with lymph node (LN) enlargement, but 70-85% of patients present with advanced clinical stage **III** or IV disease at the time of diagnosis, with a high incidence of bone marrow infiltration.

FL generally has an indolent course and initially responds well to chemotherapy. However, repeated recurrences are common in FL patients, especially in advanced stages. Analysis after the introduction of rituximab (R) showed that approximately 10% of patients underwent histologic transformation to aggressive lymphoma within five years. Data prior to the introduction of rituximab indicated that the median survival time for FL patients was 7-10 years, but recent reports suggest that the median survival time for patients aged 40 years or younger at diagnosis exceeds 20 years.

The Ann Arbor classification is used for staging in FL. Prognosis prediction models specialized for FL include the Follicular Lymphoma International Prognostic Index (FLIPI) (Non-Patent Document 1) and FLIPI2 (Non-Patent Document 2).

Cases in which disease progression or recurrence occurs within two years of diagnosis (POD24, Non-Patent Document 3) have been reported to have a poor prognosis, but no method for predicting this has been established. Among those diagnosed with FL, those with low tumor burden are actively selected for "watch and wait". Interestingly, when "watch and wait" is selected, spontaneous regression is observed in approximately 20% of cases, suggesting that the patient is receiving continuous external stimulation from the surrounding microenvironment.

### Citation List

### Non-Patent Documents

Non-Patent Document 1: Philippe Solal-Celigny and 34 others, Follicular Lymphoma International Prognostic Index, Blood, September 1, 2004, Volume 104, No. 5, p.1258-1265
Non-Patent Document 2: Massimo Federico and 19 others, Follicular Lymphoma International Prognostic Index 2: A New Prognostic Index for Follicular Lymphoma Developed by the International Follicular Lymphoma, Journal of Clinical Oncology, September 20, 2009, Vol. 27, No. 27, pp. 4555-4562, Prognostic Factor Project
Non-Patent Document 3: Carla Casulo and 11 others, Early Relapse of Follicular Lymphoma After Rituximab Plus Cyclophosphamide, Doxorubicin, Vincristine, and Prednisone Defines Patients at High Risk for Death: An Analysis From the National LymphoCare Study, Journal of Clinical Oncology, August 10, 2015, Vol. 23, No. 23, pp. 2516-2522

### SUMMARY OF INVENTION

### Technical Problem

The above-mentioned FLIPI and FLIPI2 include age as a factor and primarily reflect overall survival rate, but with improvements in anticancer drug treatment, emphasis is now being placed on recurrence rate (especially early recurrence rate within two years), and there is a need for the development of a new prognosis prediction system that is in line with the times.

Furthermore, although the above-mentioned POD24 accurately predicts the overall survival rate, this indicator itself indicates early recurrence, and since it takes 24 months for this factor to be determined, the risk at the time of diagnosis is unknown and it cannot lead to improvements in initial treatment.

The present invention has been made in view of the above circumstances, and an objective of the present invention is to provide a technique useful for predicting the prognosis in patients with follicular lymphoma.

### Solution to Problem

As a result of intensive research to solve the above-mentioned problems, the present inventors discovered that neoplastic follicle regulatory T cells (T neoplastic follicular regulatory cells: Tnfr) and neoplastic follicle cytotoxic T cells (T neoplastic follicular cytotoxic cells: Tnfc) can be detected among T cells collected from a subject by an antibody immune reaction using a specific combination of antibodies, and thus completed the present invention.

The aspects of the present invention are as follows.
[1] A method for quantifying neoplastic follicle regulatory T cells (Tnfr) and neoplastic follicle cytotoxic T cells (Tnfc) among T cells collected from a subject for predicting prognosis of follicular lymphoma, including:
   a flow cytometry step of detecting and enumerating neoplastic follicle regulatory T cells (Tnfr) and neoplastic follicle cytotoxic T cells (Tnfc) among T cells collected from a subject by a flow cytometry method using an anti-CD4 antibody, an anti-CD8a antibody, an anti-PD-1 antibody, an anti-CD25 antibody, and an anti-TIM-3 antibody; or
   a multiplex immunostaining step of detecting and enumerating neoplastic follicle regulatory T cells (Tnfr) and neoplastic follicle cytotoxic T cells (Tnfc) among T cells collected from a subject by a multiplex immunoassay using an anti-CD4 antibody, an anti-CD8 antibody, an anti-PD-1 antibody, an anti-FOXP3 antibody, an anti-TCF-1 antibody, and an anti-Granzyme K antibody.
[2] The method according to [1], wherein in the flow cytometry step, CD4⁺ PD-1⁺ CD25⁺ cells are detected and enumerated as neoplastic follicle regulatory T cells (Tnfr), and CD8a⁺ PD-1⁺ TIM-3⁻ cells are detected and enumerated as CD8-positive neoplastic follicle cytotoxic T cells (Tnfc8).
[3] The method according to [1], wherein in the multiplex immunostaining step, CD4⁺ PD-1⁺ FOXP3⁺ cells are detected and enumerated as neoplastic follicle regulatory T cells (Tnfr), CD8⁺ Granzyme K⁺ PD-1⁺ TCF-1⁺ cells are detected and enumerated as CD8-positive neoplastic follicle cytotoxic T cells (Tnfc8), and CD4⁺ Granzyme K⁺ PD-1⁺ TCF-1⁺ cells are detected and enumerated as CD4-positive neoplastic follicle cytotoxic T cells (Tnfc4).
[4] A method for screening neoplastic follicle regulatory T cells (Tnfr) and neoplastic follicle cytotoxic T cells (Tnfc) from T cells collected from a subject for predicting prognosis of follicular lymphoma, including:
   a flow cytometry step of detecting and enumerating neoplastic follicle regulatory T cells (Tnfr) and neoplastic follicle cytotoxic T cells (Tnfc) among T cells collected from a subject by a flow cytometry method using an anti-CD4 antibody, an anti-CD8a antibody, an anti-PD-1 antibody, an anti-CD25 antibody, and an anti-TIM-3 antibody; or
   a multiplex immunostaining step of detecting and enumerating neoplastic follicle regulatory T cells (Tnfr) and neoplastic follicle cytotoxic T cells (Tnfc) among T cells collected from a subject by a multiplex immunoassay using an anti-CD4 antibody, an anti-CD8 antibody, an anti-PD-1 antibody, an anti-FOXP3 antibody, an anti-TCF-1 antibody, and an anti-Granzyme K antibody.
[5] The method according to [4], wherein in the flow cytometry step, CD4⁺ PD-1⁺ CD25⁺ cells are detected and enumerated as neoplastic follicle regulatory T cells (Tnfr), and CD8a⁺ PD-1⁺ TIM-3⁻ cells are detected and enumerated as CD8-positive neoplastic follicle cytotoxic T cells (Tnfc8).
[6] The method according to [4], wherein in the multiplex immunostaining step, CD4⁺ PD-1⁺ FOXP3⁺ cells are detected and enumerated as neoplastic follicle regulatory T cells (Tnfr), CD8⁺ Granzyme K⁺ PD-1⁺ TCF-1⁺ cells are detected and enumerated as CD8-positive neoplastic follicle cytotoxic T cells (Tnfc8), and CD4⁺ Granzyme K⁺ PD-1⁺ TCF-1⁺ cells are detected and enumerated as CD4-positive neoplastic follicle cytotoxic T cells (Tnfc4).
[7] A therapeutic composition for treating follicular lymphoma, including T cells detected as CD4⁺ PD-1⁺ CD25⁺ cells or CD8a⁺ PD-1⁺ TIM-3⁻ cells in a flow cytometry step of detecting and enumerating neoplastic follicle regulatory T cells (Tnfr) and neoplastic follicle cytotoxic T cells (Tnfc) among T cells collected from a subject by a flow cytometry method using an anti-CD4 antibody, an anti-CD8a antibody, an anti-PD-1 antibody, an anti-CD25 antibody, and an anti-TIM-3 antibody.
[8] A therapeutic composition for treating follicular lymphoma, including T cells detected as CD4⁺ PD-1⁺ FOXP3⁺ cells, CD8⁺ Granzyme K⁺ PD-1⁺ TCF-1⁺ cells, or CD4⁺ Granzyme K⁺ PD-1⁺ TCF-1⁺ cells in a multiplex immunostaining step of detecting and enumerating neoplastic follicle regulatory T cells (Tnfr) and neoplastic follicle cytotoxic T cells (Tnfc) among T cells collected from a subject by a multiplex immunoassay using an anti-CD4 antibody, an anti-CD8 antibody, an anti-PD-1 antibody, an anti-FOXP3 antibody, an anti-TCF-1 antibody, and an anti-Granzyme K antibody.
[9] The therapeutic composition according to [7] or [8], wherein the T cells are T cells pre-cultured with IL-21.
[10] The therapeutic composition according to [7] or [8], wherein the T cells are artificially genetically modified T cells.
[11] A kit for predicting prognosis of follicular lymphoma, including:
   an antibody set including an anti-CD4 antibody, an anti-CD8a antibody, an anti-PD-1 antibody, an anti-CD25 antibody, and an anti-TIM-3 antibody; or
   an antibody set including an anti-CD4 antibody, an anti-CD8 antibody, an anti-PD-1 antibody, an anti-FOXP3 antibody, an anti-TCF-1 antibody, and an anti-Granzyme K antibody.
[12] The kit according to [11], which is for identifying, detecting, or screening neoplastic follicle regulatory T cells (Tnfr) and neoplastic follicle cytotoxic T cells (Tnfc) from a population of T cells collected from a subject.
[13] A method for assisting in predicting prognosis of follicular lymphoma in a subject, including:
   a flow cytometry step of detecting and enumerating neoplastic follicle regulatory T cells (Tnfr) and neoplastic follicle cytotoxic T cells (Tnfc) among T cells collected from a subject by a flow cytometry method using an anti-CD4 antibody, an anti-CD8a antibody, an anti-PD-1 antibody, an anti-CD25 antibody, and an anti-TIM-3 antibody; and
   a ratio calculation step of calculating ratios of neoplastic follicle regulatory T cells (Tnfr), CD4-positive neoplastic follicle cytotoxic T cells (Tnfc4), and CD8-positive neoplastic follicle cytotoxic T cells (Tnfc8) among the T cells collected from the subject.
[14] The method according to [13], wherein in the ratio calculation step, the ratio of CD8-positive neoplastic follicle cytotoxic T cells (Tnfc8) is calculated based on results of detecting and enumerating CD4-positive neoplastic follicle cytotoxic T cells (Tnfc4).
[15] A method for assisting in predicting prognosis of follicular lymphoma in a subject, including:
   a multiplex immunostaining step of detecting and enumerating neoplastic follicle regulatory T cells (Tnfr) and neoplastic follicle cytotoxic T cells (Tnfc) among T cells collected from a subject by a multiplex immunoassay using an anti-CD4 antibody, an anti-CD8 antibody, an anti-PD-1 antibody, an anti-FOXP3 antibody, an anti-TCF-1 antibody, and an anti-Granzyme K antibody; and
   a ratio calculation step of calculating ratios of neoplastic follicle regulatory T cells (Tnfr), CD4-positive neoplastic follicle cytotoxic T cells (Tnfc4), and CD8-positive neoplastic follicle cytotoxic T cells (Tnfc8) among the T cells collected from the subject.
[16] A method for predicting prognosis of follicular lymphoma in a subject, including:
   a quantifying step of quantifying neoplastic follicle regulatory T cells (Tnfr) and neoplastic follicle cytotoxic T cells (Tnfc) among T cells collected from a subject by the method according to any one of [1] to [6];
   a ratio calculation step of calculating ratios of neoplastic follicle regulatory T cells (Tnfr), CD4-positive neoplastic follicle cytotoxic T cells (Tnfc), and CD8-positive neoplastic follicle cytotoxic T cells (Tnfc) among the T cells collected from the subject based on quantification results obtained in the quantification step; and
   a prognosis evaluation step of evaluating the prognosis of the subject based on the ratios calculated in the ratio calculation step, wherein
   in the prognosis evaluation step, if the ratios calculated in the ratio calculation step are less than 2.0% for neoplastic follicle regulatory T cells (Tnfr) and less than 2.5% for a sum of the CD4-positive neoplastic follicle cytotoxic T cells (Tnfc4) and the CD8-positive neoplastic follicle cytotoxic T cells (Tnfc8), the prognosis is assessed as poor.

### Advantageous Effects of Invention

According to the present invention, a technique useful for predicting the prognosis of patients with follicular lymphoma can be provided.

### BRIEF DESCRIPTION OF DRAWINGS

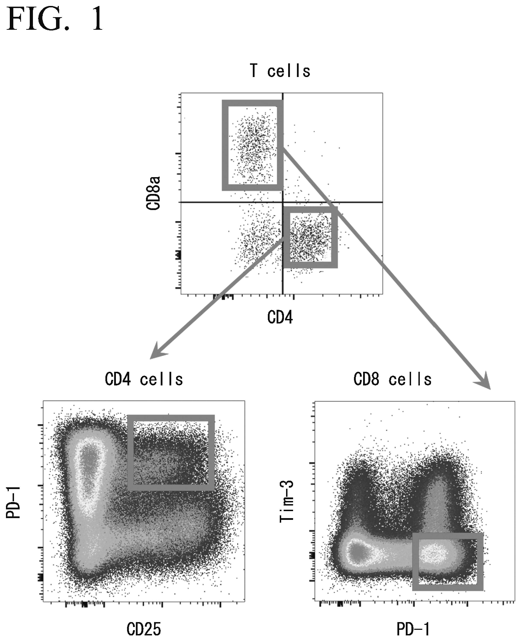

[FIG. 1] FIG. 1 shows the results of Experimental Example 1. The graph shows the results of flow cytometry.

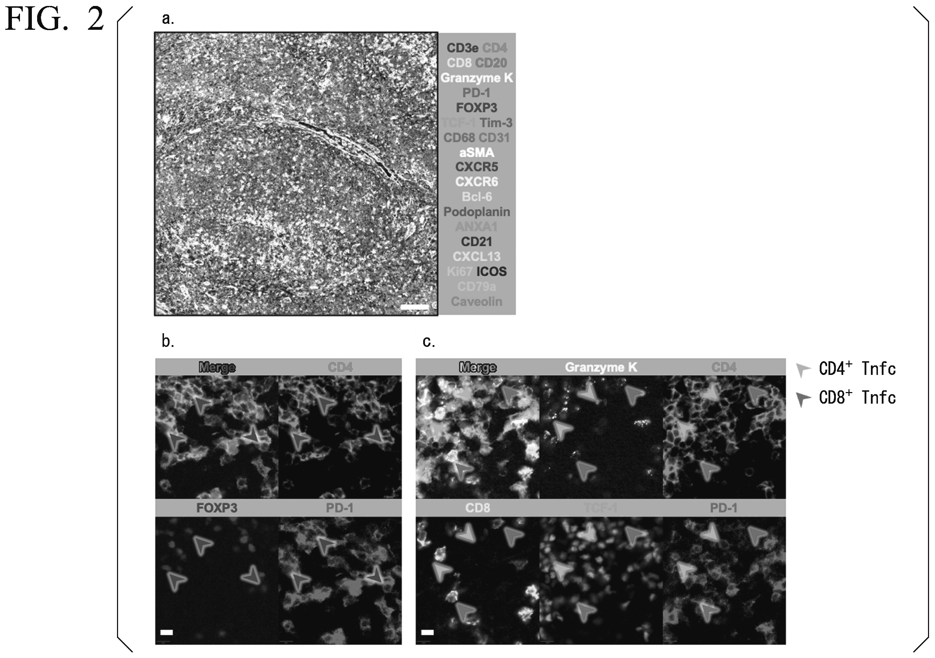

[FIG. 2] FIG. 2 shows the results of Experimental Example 2. The images show the results of multiplex immunostaining: a. Multiplex immunostaining; b. Detection of Tnfr; c. Detection of Tnfc.

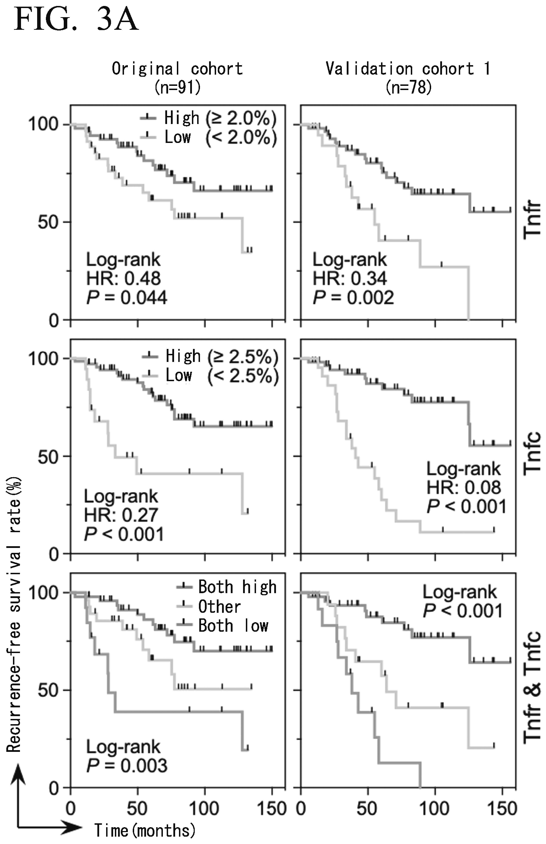

[FIG. 3A] FIG. 3A shows the results of Experimental Example 3. The graph shows the relationship between the recurrence rate (vertical axis) and the time period (horizontal axis) for follicular lymphoma patient cohorts (original cohort and validation cohort 1) according to the ratio of neoplastic follicle regulatory T cells (Tnfr) and neoplastic follicle cytotoxic T cells (Tnfc).

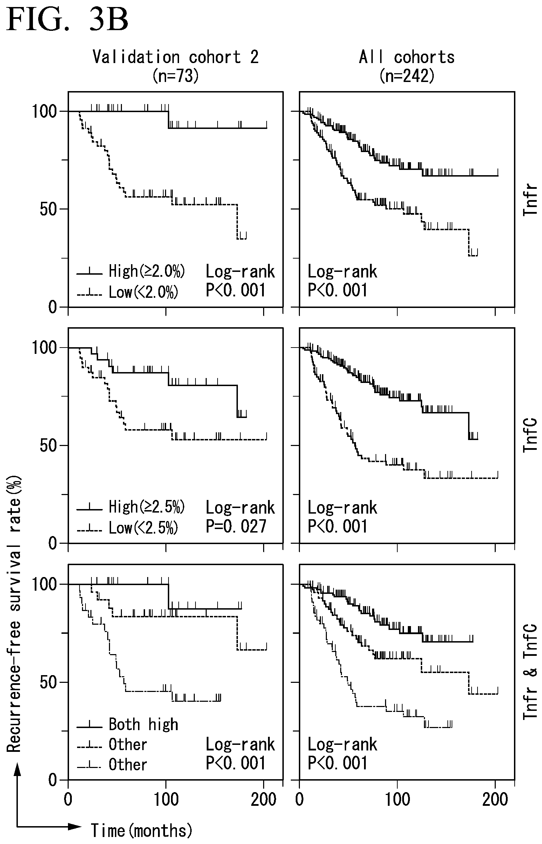

[FIG. 3B] FIG. 3B shows the results of Experimental Example 3. The graph shows the relationship between the recurrence rate (vertical axis) and the time period (horizontal axis) for follicular lymphoma patient cohorts (validation cohort 2 and all cohorts) according to the ratio of neoplastic follicle regulatory T cells (Tnfr) and neoplastic follicle cytotoxic T cells (Tnfc).

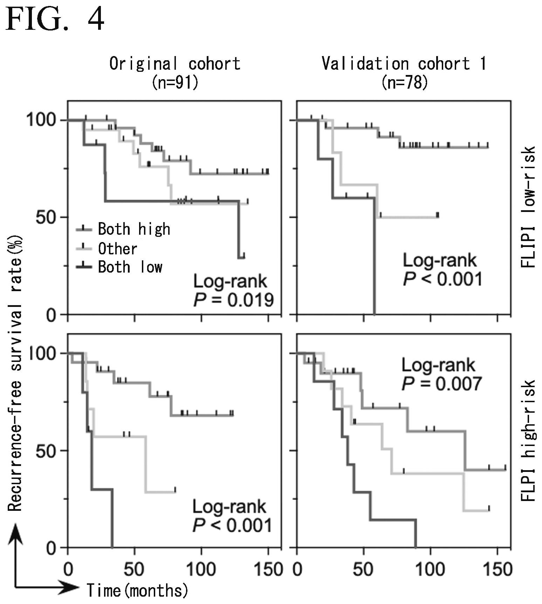

[FIG. 4] FIG. 4 shows the results of Experimental Example 3. The graph shows the relationship between the recurrence rate (vertical axis) and the period (horizontal axis) for follicular lymphoma patient cohorts judged to be low risk and follicular lymphoma patient cohorts judged to be high risk according to risk classification by FLIPI (original cohort and validation cohort 1).

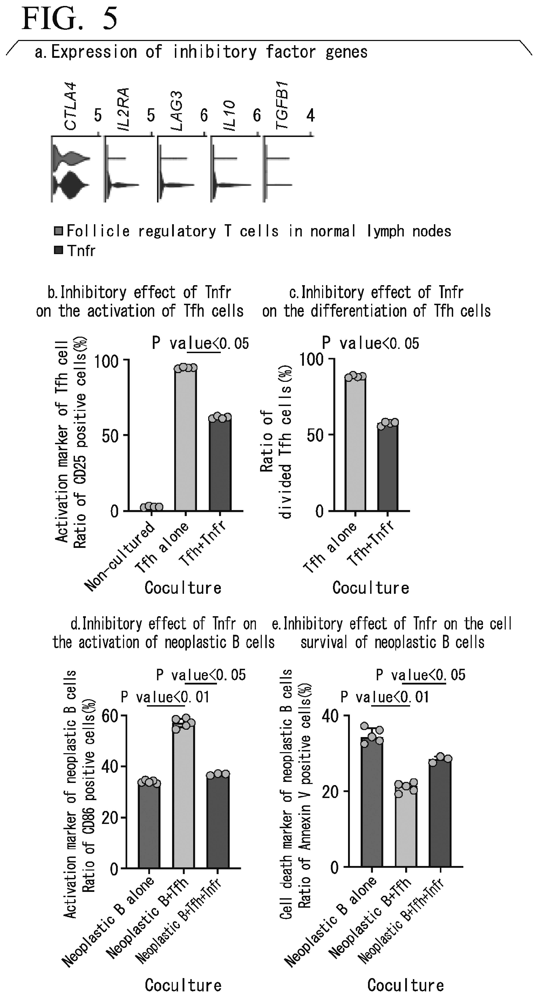

[FIG. 5] FIG. 5 shows the results of Experimental Example 4: a. Violin plots of inhibitory factor gene expression in Tnfr and normal lymph node from follicle regulatory T cells; b. Inhibitory effect of Tnfr on the activation of Tfh cells; c. Inhibitory effect of Tnfr on the differentiation of Tfh cells; d. Inhibitory effect of Tnfr on the activation of neoplastic B cells; e. Inhibitory effect of Tnfr on the cell survival of neoplastic B cells.

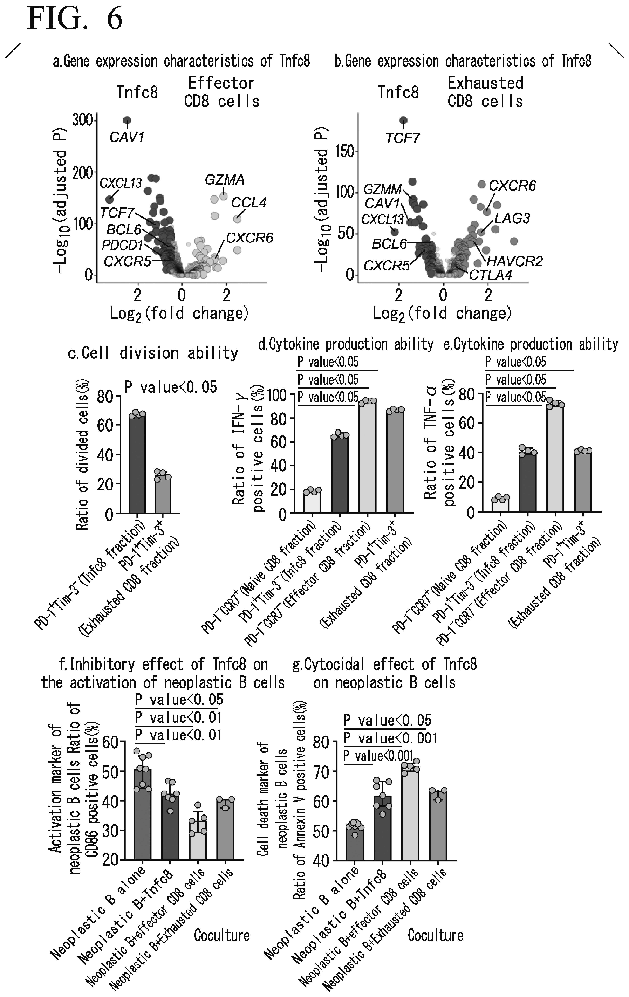

[FIG. 6] FIG. 6 shows the results of Experimental Example 5: a. Comparison of gene expression between Tnfc8 and effector CD8 cells; b. Comparison of gene expression between Tnfc8 and exhausted CD8 cells; c. Cell division ability; d. Interferon gamma production ability; e. TNF alpha production ability; f. Inhibitory effect of Tnfc8 on the activation of neoplastic B cells; g. Cytocidal effect of Tnfc8 on neoplastic B cells.

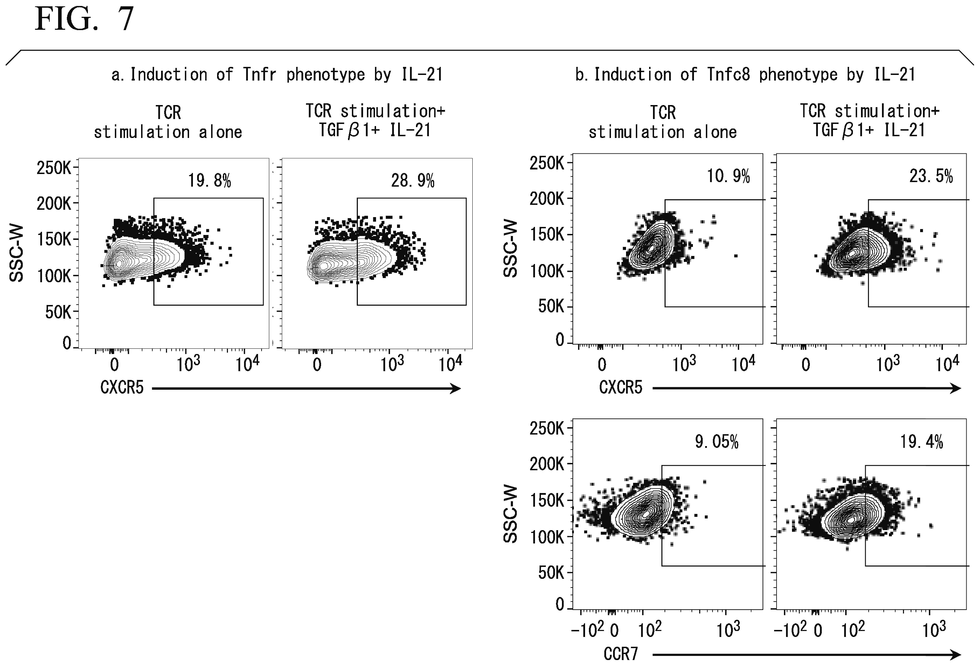

[FIG. 7] FIG. 7 shows the results of culturing each type of cell line under T cell receptor (TCR) stimulation in the presence of transforming growth factor (TGF) β1, with or without the addition of IL-21, in Experimental Example 6. a. Induction of Tnfr phenotype by IL-21; b. Induction of Tnfc8 phenotype by IL-21.

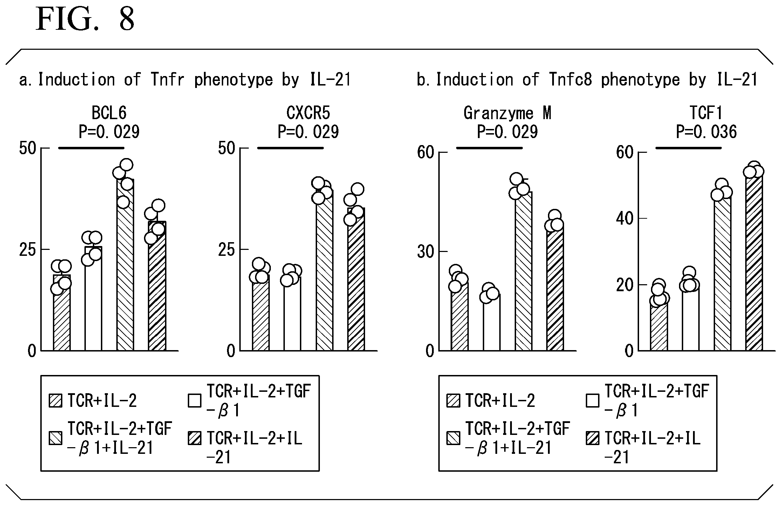

[FIG. 8] FIG. 8 shows the results of culturing each type of cell line under TCR stimulation with or without the addition of TGFβ1 and IL-21 in Experimental Example 6. a. Induction of Tnfr phenotype by IL-21; b. Induction of Tnfc8 phenotype by IL-21.

### DESCRIPTION OF EMBODIMENTS

In the present invention, when a numerical range is expressed using "to", the numerical range includes the numerical values before and after "to".

In the present invention, the "subject" refers to a human from whom T cells are to be collected, and is preferably a human suffering from follicular lymphoma.

The following describes in detail the embodiments of the present invention. However, the present invention is not limited to the embodiments described below, and various modifications are possible as long as they do not depart from the scope of the present invention.

### [Quantitative method]

The method of the present invention for quantifying neoplastic follicle regulatory T cells and neoplastic follicle cytotoxic T cells among T cells collected from a subject (hereinafter also simply referred to as "quantification method") includes a flow cytometry step or a multiplex immunostaining step.

The flow cytometry step and the multiplex immunostaining step are steps carried out in vitro.

### <Flow cytometry process>

The flow cytometry step is a step of detecting and enumerating neoplastic follicle regulatory T cells (Tnfr) and neoplastic follicle cytotoxic T cells (Tnfc) among T cells collected from a subject by a flow cytometry method using an antibody set including an anti-CD4 (cluster of differentiation 4) antibody, an anti-CD8a antibody, an anti-PD-1 (programmed cell death protein 1) antibody, an anti-CD25 antibody, and an anti-TIM-3 (T cell immunoglobulin domain and mucin domain 3) antibody.

The flow cytometry method can be a method conventionally used in the art, except that the above-mentioned antibody set is used as the antibody for detecting cells.

The aforementioned antibody may be a primary antibody that is itself fluorescently labeled, metal-labeled, or DNA barcode-labeled, or may be an antibody to which a secondary antibody that is fluorescently labeled, metal-labeled, or DNA barcode-labeled specifically binds.

Common progenitor cells derived from bone marrow differentiate into three types of T cells with different functions in the thymus. One type expresses γδ receptors, which do not recognize peptide antigens presented by MHC (Major Histocompatibility Complex) molecules, and the other two types express αβ receptors. αβ-type T cells undergo positive and negative selection in the thymus, and differentiate into CD4 or CD8 single-positive cells, depending on the specificity of the receptor for MHC. CD4 and CD8 are coreceptors expressed on the surface of T cells and bind to conserved regions of MHC class II molecules and MHC class I molecules, respectively.

When PD-1 expressed on activated T cells binds to PD-L1 or PD-L2 expressed on cancer cells or antigen-presenting cells, T cell activation is inhibited, causing the cancer cells to escape the immune system. Anti-PD-1 antibodies bind to PD-1 on T cells and inhibit the binding of PD-1 to PD-L1/PD-L2, thereby blocking the transmission of inhibitory signals, maintaining T cell activation and restoring antitumor effects.

The CD25 (IL-2Rα chain, Tac or p55) antigen is a 55-kDa monosaccharide protein and a low-affinity receptor for IL-2 (IL-2Rα or IL-2R1). It forms the high-affinity IL-2R complex with CD12 (IL-2Rβ chain, p75) and CD132 (IL-2Rγ chain, p64). It is highly expressed on activated regulatory CD4-positive T cells but not on resting CD8-positive lymphocytes. However, all activated T cells express the CD25 protein. CD25 is also found on a subset of B cells (CD20+), activated monocytes, macrophages, and T-cell clones. A recombinant human/mouse chimeric anti-CD25 monoclonal antibody (IgG1) is known as basiliximab (recombinant).

TIM-3 (also known as HAVCR2) is a recently discovered member of the T cell Ig and mucin domain containing molecule superfamily. TIM-3 is a key regulatory molecule in T cell tolerance and plays a crucial role in autoimmunity and T cell exhaustion during chronic viral infection. TIM-3 is expressed on Th1 cells (type 1 T helper cells), macrophages, monocytes, dendritic cells, CD8-positive T cells, and other lymphocyte subsets. The ligand for TIM-3 is galectin-9. The TIM-3-galectin-9 pathway negatively regulates Th1 cell (type 1 T helper cell) immunity. TIM3 is a membrane protein consisting of 280 residues with a calculated molecular weight of 33 kDa.

The antibody set described above may further include an anti-CD3 antibody. The anti-CD3 antibody recognizes the human CD3 molecule. CD3 is a component of the T cell receptor and is most commonly used as a T cell marker. As the anti-CD3 antibody, for example, an anti-CD3e antibody that recognizes the ε chain of the human CD3 molecule may be used.

In the flow cytometry step, neoplastic follicle regulatory T cells (Tnfr) are detected and enumerated as CD4⁺ PD-1⁺ CD25⁺ cells.

In the flow cytometry step, CD8-positive neoplastic follicle cytotoxic T cells (Tnfc8) are detected and enumerated as CD8a⁺ PD-1⁺ TIM-3⁻ cells.

In the flow cytometry step, CD4-positive neoplastic follicle cytotoxic T cells (Tnfc4) are not directly detected and enumerated, but there is a strong correlation between the ratios of CD8-positive neoplastic follicle cytotoxic T cells and CD4-positive neoplastic follicle cytotoxic T cells, making it possible to calculate the ratio of CD4-positive neoplastic follicle cytotoxic T cells. Specifically, as shown in the Experimental Examples described below, a strong positive correlation was observed in a study of 169 patients, with a correlation coefficient (Spearman's) of 0.93.

### <Multiplex immunostaining step>

The multiplex immunostaining step is a step of detecting and enumerating neoplastic follicle regulatory T cells (Tnfr) and neoplastic follicle cytotoxic T cells (Tnfc) among T cells collected from a subject by a multiplex immunoassay using an anti-CD4 antibody, an anti-CD8 antibody, an anti-PD-1 antibody, an anti-FOXP3 (forkhead box P3) antibody, an anti-TCF-1 (T cell factor 1) antibody, and an anti-Granzyme K antibody.

The multiplex immunostaining method can be a method conventionally used in the art, except that the above-mentioned antibody set is used as the antibody for detecting cells.

The anti-CD4 antibody and anti-PD-1 antibody are as described above.

The aforementioned antibody may be a primary antibody that is itself fluorescently labeled, metal-labeled, or DNA barcode-labeled, or may be an antibody to which a secondary antibody that is fluorescently labeled, metal-labeled, or DNA barcode-labeled specifically binds.

The transcription factor T cell factor 1 (TCF-1) is essential for T cell development in the thymus, as well as a downstream effector of WNT/-catenin signaling.

The FOXP3 molecule is a 50-55 kD transcription factor, also known as IPEX, JM2, Forkhead box P3, and Scurfin.

In the multiplex immunostaining step, neoplastic follicle regulatory T cells (Tnfr) are detected and enumerated as CD4⁺ PD-1⁺ FOXP3⁺ cells.

In the multiplex immunostaining step, CD8-positive neoplastic follicle cytotoxic T cells (Tnfc8) are detected and enumerated as CD8⁺ Granzyme K⁺ PD-1⁺ TCF-1⁺ cells.

In the multiplex immunostaining step, CD4-positive neoplastic follicle cytotoxic T cells (Tnfc4) are detected and enumerated as CD4⁺ Granzyme K⁺ PD-1⁺ TCF-1⁺ cells.

### [Screening method]

The method of the present invention for screening neoplastic follicle regulatory T cells (Tnfr) and neoplastic follicle cytotoxic T cells (Tnfc) from T cells collected from a subject (hereinafter also simply referred to as the "screening method") includes a flow cytometry step or a multiplex immunostaining step.

The flow cytometry step and the multiplex immunostaining step are the same as those **in** the above-mentioned quantification method.

In the flow cytometry step, neoplastic follicle regulatory T cells (Tnfr) are screened as CD4⁺ PD-1⁺ CD25⁺ cells, and CD8-positive neoplastic follicle cytotoxic T cells (Tnfc8) are screened as CD8a⁺ PD-1⁺ TIM-3⁻ cells.

In the multiplex immunostaining step, neoplastic follicle regulatory T cells (Tnfr) are screened as CD4⁺ PD-1⁺ FOXP3⁺ cells, CD8-positive neoplastic follicle cytotoxic T cells (Tnfc8) are screened as CD8⁺ Granzyme K⁺ PD-1⁺ TCF-1⁺ cells, and CD4-positive neoplastic follicle cytotoxic T cells (Tnfc4) are screened as CD4⁺ Granzyme K⁺ PD-1⁺ TCF-1⁺ cells.

### [T cells]

In an embodiment of the T cells of the present invention, the T cells are detected as CD4⁺ PD-1⁺ CD25⁺ cells or CD8a⁺ PD-1⁺ TIM-3⁻ cells in a flow cytometry step of detecting and enumerating neoplastic follicle regulatory T cells (Tnfr) and neoplastic follicle cytotoxic T cells (Tnfc) among T cells collected from a subject by a flow cytometry method using an anti-CD4 antibody, an anti-CD8 antibody, an anti-PD-1 antibody, an anti-CD25 antibody, and an anti-TIM-3 antibody.

The flow cytometry process is similar to the flow cytometry process described above.

In another embodiment of the T cells of the present invention, the T cells are detected as CD4⁺ PD-1⁺ FOXP3⁺ cells, CD8⁺ Granzyme K⁺ PD-1⁺ TCF-1⁺ cells, or CD4⁺ Granzyme K⁺ PD-1⁺ TCF-1⁺ cells in a multiplex immunostaining step of detecting and enumerating neoplastic follicle regulatory T cells (Tnfr) and neoplastic follicle cytotoxic T cells (Tnfc) among T cells collected from a subject by a multiplex immunoassay using an anti-CD4 antibody, an anti-CD8 antibody, an anti-PD-1 antibody, an anti-FOXP3 antibody, an anti-TCF-1 antibody, and an anti-Granzyme K antibody.

The multiplex immunostaining step is the same as the multiplex immunostaining step described above.

The T cells of the present embodiment can be used to treat follicular lymphoma.

As shown in Experimental Examples 5 to 7 described below, Tnfr has an inhibitory effect on the activation and differentiation of follicle helper T cells (Tfh) in normal lymph nodes, while Tnfc has an activation-inhibitory effect and cytocidal effect on neoplastic B cells, which are cancer cells in follicular lymphoma.

Furthermore, Tnfr has increased expression of inhibitory genes such as IL2RA, LAG3, and IL10, while Tnfc highly expresses CXCL13, CXCR5, and BCL6 genes, which are factors that promote migration and settlement into follicular structures, as well as the TCF7 gene, which maintains the cellular stemness.

Furthermore, the cytokine IL-21 can induce Tnfr cells from regulatory T cells (high expression of CXCR5 and BCL6), and can induce Tnfc8 cells from naive CD8 cells (high expression of CXCR5, CCR7, Granzyme M, and TCF1). That is, the T cells of the present embodiment are preferably T cells pre-cultured with IL-21. Through the cell induction method, adding the characteristics of Tnfr and Tnfc cells to T cell genetic engineering therapies is expected to improve therapeutic efficacy.

Neoplastic follicle regulatory T cells (Tnfr) and neoplastic follicle cytotoxic T cells (Tnfc) extracted from patient T cells can be used to develop therapeutics for use as direct treatments.

In one embodiment of the T cells of the present invention, the T cells of the above-mentioned embodiment or the T cells of the above-mentioned other embodiment may be further artificially genetically modified.

In the present invention, "artificial genetic modification" means modifying the function of T cells by gene introduction into T cells or gene editing of T cells. The "artificial genetic modification" in the present invention may be a modification of a gene possessed by the T cell itself, a deletion of a gene possessed by the T cell itself, a modification in which a foreign gene has been introduced, or a combination of two or more of these, and is preferably a modification in which a foreign gene has been introduced.

Methods for artificial gene modification include, but are not limited to, conventionally known methods and methods based thereon, such as a method using a viral vector, agroinfection, calcium phosphate method, microinjection method, particle gun method, DEAE-dextran method, electroporation method, cationic lipid method, and gene editing (genome editing) of T cells using site-specific nucleases (meganuclease, zinc finger nuclease, TALEN, PPR, CRISPR-Cas, etc.).

The artificial gene modification method may be one of these methods alone or a combination of two or more of these methods.

The gene that alters the function of a T cell, i.e., the gene to be altered by artificial genetic modification, is preferably at least one selected from the group consisting of genes encoding a fusion protein including a protein expressed on the surface of a T cell and at least one intracellular signaling domain, and genes encoding a fusion protein including a protein expressed on the surface of a T cell, at least one costimulatory domain, and at least one intracellular signaling domain, more preferably genes encoding an antigen receptor, even more preferably at least one selected from the group consisting of genes encoding a T cell receptor (TCR) and genes encoding a chimeric antigen receptor (CAR), and particularly preferably genes encoding a chimeric antigen receptor (CAR).

That is, in one embodiment of the T cells of the present invention, it is preferable that the T cells express on their surface a surface protein encoded by a gene of interest that has been modified by artificial genetic modification. Such genetically modified T cells are more preferably at least one type selected from the group consisting of TCR-T cells that express a T cell receptor (TCR) encoded by a gene that has been modified by artificial genetic modification, and CAR-T cells that express a chimeric antigen receptor (CAR), and are even more preferably CAR-T cells.

### [Therapeutic composition]

One embodiment of the therapeutic composition of the present invention is a therapeutic composition for treating follicular lymphoma, including T cells that are detected as CD4⁺ PD-1⁺ CD25⁺ cells or CD8a⁺ PD-1⁺ TIM-3⁻ cells in a flow cytometry step of detecting and enumerating neoplastic follicle regulatory T cells and neoplastic follicle cytotoxic T cells among T cells collected from a subject by a flow cytometry method using an anti-CD4 antibody, an anti-CD8a antibody, an anti-PD-1 antibody, an anti-CD25 antibody, and an anti-TIM-3 antibody.

The T cells are the same as the T cells of the present embodiment.

Alternatively, another embodiment of the therapeutic composition of the present invention is a therapeutic composition for treating follicular lymphoma, including T cells detected as CD4⁺ PD-1⁺ FOXP3⁺ cells, CD8⁺ Granzyme K⁺ PD-1⁺ TCF-1⁺ cells, or CD4⁺ Granzyme K⁺ PD-1⁺ TCF-1⁺ cells in a multiplex immunostaining step of detecting and enumerating neoplastic follicle regulatory T cells and neoplastic follicle cytotoxic T cells among T cells collected from a subject by a multiplex immunoassay using an anti-CD4 antibody, an anti-CD8 antibody, an anti-PD-1 antibody, an anti-FOXP3 antibody, an anti-TCF-1 antibody, and an anti-Granzyme K antibody.

The T cells are the same as the T cells of the present embodiment.

The therapeutic composition of the embodiment may contain T cells obtained by further culturing and expanding the T cells of the present embodiment, or by concentrating the T cells by centrifugation or the like, or by an appropriate combination of these methods, and may contain, in addition to the T cells of the present embodiment, known culture media, cell washing solutions, excipients, additives, and the like.

The T cells of the present embodiment can be used to manufacture a therapeutic composition for treating follicular lymphoma.

The therapeutic composition of the present embodiment can be formulated into an injection or the like by a conventional method together with various additives, and used to produce a therapeutic agent for follicular lymphoma.

**In** one embodiment, the present invention may be a method for treating a patient with follicular lymphoma using the T cells or therapeutic composition of the present embodiment. The treatment method may be a method conventionally performed in the technical field, except that the T cells or therapeutic composition of the present embodiment is used. For example, the treatment method may be chimeric antigen receptor-T cell therapy (CAR-T cell therapy) using the T cells or therapeutic composition of the present embodiment, T cell receptor (TCR)-T cell therapy (TCR-T cell therapy) using the T cells or therapeutic composition of the present embodiment, or tumor-infiltrating lymphocyte therapy (TIL therapy) using the T cells or therapeutic composition of the present embodiment.

CAR-T cell therapy using the T cells or therapeutic composition of the present embodiment may be a treatment method for patients with B-cell acute lymphoblastic leukemia, diffuse large B-cell lymphoma, multiple myeloma, chronic lymphocytic leukemia, acute myeloid leukemia, or the like.

TCR-T cell therapy using the T cells or therapeutic composition of the present embodiment may be a treatment method for patients with malignant melanoma, ovarian cancer, non-small cell lung cancer, esophageal cancer, colorectal cancer, synovial sarcoma, myeloma, osteosarcoma, malignant mesothelioma, adult T- cell leukemia/lymphoma, myelodysplastic syndrome, acute myeloid leukemia, or the like.

TIL therapy using the T cells or therapeutic composition of the present embodiment may be a treatment method for patients with malignant melanoma, cervical cancer, and the like.

That is, the present invention may include the following aspects:
a method for treating a patient with follicular lymphoma, including:
using T cells detected as CD4⁺ PD-1⁺ CD25⁺ cells or CD8a⁺ PD-1⁺ TIM-3⁻ cells in a flow cytometry step of detecting and enumerating neoplastic follicle regulatory T cells (Tnfr) and neoplastic follicle cytotoxic T cells (Tnfc) among T cells collected from a subject by a flow cytometry method using an anti-CD4 antibody, an anti-CD8 antibody, an anti-PD-1 antibody, an anti-CD25 antibody, and an anti-TIM-3 antibody;
   or
a method for treating a patient with follicular lymphoma, including:
   using T cells detected as CD4⁺ PD-1⁺ FOXP3⁺ cells, CD8⁺ Granzyme K⁺ PD-1⁺ TCF-1⁺ cells, or CD4⁺ Granzyme K⁺ PD-1⁺ TCF-1⁺ cells in a multiplex immunostaining step of detecting and enumerating neoplastic follicle regulatory T cells (Tnfr) and neoplastic follicle cytotoxic T cells (Tnfc) among T cells collected from a subject by a multiplex immunoassay using an anti-CD4 antibody, an anti-CD8 antibody, an anti-PD-1 antibody, an anti-FOXP3 antibody, an anti-TCF-1 antibody, and an anti-Granzyme K antibody;
      or
   a method for treating a patient with follicular lymphoma, including:
      using a therapeutic composition including T cells detected as CD4⁺ PD-1⁺ CD25⁺ cells or CD8a⁺ PD-1⁺ TIM-3⁻ cells in a flow cytometry step of detecting and enumerating neoplastic follicle regulatory T cells (Tnfr) and neoplastic follicle cytotoxic T cells (Tnfc) among T cells collected from a subject by a flow cytometry method using an anti-CD4 antibody, an anti-CD8 antibody, an anti-PD-1 antibody, an anti-CD25 antibody, and an anti-TIM-3 antibody;
         or
      a method for treating a patient with follicular lymphoma, including:
         using a therapeutic composition including T cells detected as CD4⁺ PD-1⁺ FOXP3⁺ cells, CD8⁺ Granzyme K⁺ PD-1⁺ TCF-1⁺ cells, or CD4⁺ Granzyme K⁺ PD-1⁺ TCF-1⁺ cells in a multiplex immunostaining step of detecting and enumerating neoplastic follicle regulatory T cells (Tnfr) and neoplastic follicle cytotoxic T cells (Tnfc) among T cells collected from a subject by a multiplex immunoassay using an anti-CD4 antibody, an anti-CD8 antibody, an anti-PD-1 antibody, an anti-FOXP3 antibody, an anti-TCF-1 antibody, and an anti-Granzyme K antibody.

### [Kit]

The kit of the present invention includes an antibody set (first antibody set) including an anti-CD4 antibody, an anti-CD8a antibody, an anti-PD-1 antibody, an anti-CD25 antibody, and an anti-TIM-3 antibody, or an antibody set (second antibody set) including an anti-CD4 antibody, an anti-CD8 antibody, an anti-PD-1 antibody, an anti-FOXP3 antibody, an anti-TCF-1 antibody, and an anti-Granzyme K antibody.

Both the first antibody set and the second antibody set described above may further include an anti-CD3 antibody or an anti-CD3e antibody.

The kit of the present invention can detect and enumerate neoplastic follicle regulatory T cells (Tnfr) and neoplastic follicle cytotoxic T cells (Tnfc) by a flow cytometry method using the first antibody set.

The kit of the present invention can detect and enumerate neoplastic follicle regulatory T cells (Tnfr) and neoplastic follicle cytotoxic T cells (Tnfc) by a multiplex immunostaining method using the second antibody set.

The kit of the present invention may further include other components, such as an experimental protocol.

The kit of the present invention can be used to diagnose or predict the prognosis of follicular lymphoma.

The kit of the present invention can be used to identify, detect, or screen for neoplastic follicle regulatory T cells (Tnfr) and neoplastic follicle cytotoxic T cells (Tnfc) from a population of T cells collected from a subject.

In one embodiment, the present invention provides the first set of antibodies and the second set of antibodies for predicting the prognosis of follicular lymphoma.

In one embodiment, the present invention provides use of the first set of antibodies and the second set of antibodies for producing a kit for predicting the prognosis of follicular lymphoma.

### [Method for assisting in prognosis prediction]

One embodiment of the method of the present invention for assisting **in** predicting the prognosis of follicular lymphoma **in** a subject (hereinafter also simply referred to as a "method for assisting **in** prognosis prediction") includes a flow cytometry step of detecting and enumerating neoplastic follicle regulatory T cells (Tnfr) and neoplastic follicle cytotoxic T cells (Tnfc) among T cells collected from the subject by a flow cytometry method using an anti-CD4 antibody, an anti-CD8a antibody, an anti-PD-1 antibody, an anti-CD25 antibody, and an anti-TIM-3 antibody, and a ratio calculation step of calculating the ratios of neoplastic follicle regulatory T cells (Tnfr), CD4-positive neoplastic follicle cytotoxic T cells (Tnfc4), and CD8-positive neoplastic follicle cytotoxic T cells (Tnfc8) among the T cells collected from the subject.

The flow cytometry step and the ratio calculation step are steps carried out in vitro.

In the ratio calculation step, it is preferable to calculate the ratio of CD8-positive neoplastic follicle cytotoxic T cells (Tnfc8) based on the results of detecting and enumerating CD4-positive neoplastic follicle cytotoxic T cells (Tnfc4).

Another embodiment of the method for assisting in prognosis prediction of the present invention is a method for assisting in predicting the prognosis of follicular lymphoma in a subject, including: a multiplex immunostaining step of detecting and enumerating neoplastic follicle regulatory T cells (Tnfr) and neoplastic follicle cytotoxic T cells (Tnfc) among T cells collected from a subject by a multiplex immunoassay using an anti-CD4 antibody, an anti-CD8 antibody, an anti-PD-1 antibody, an anti-FOXP3 antibody, an anti-TCF-1 antibody, and an anti-Granzyme K antibody; and a ratio calculation step of calculating the ratios of neoplastic follicle regulatory T cells (Tnfr), CD4-positive neoplastic follicle cytotoxic T cells (Tnfc4), and CD8-positive neoplastic follicle cytotoxic T cells (Tnfc8) among the T cells collected from the subject.

### [Method for prognosis prediction]

The method for predicting the prognosis of follicular lymphoma in a subject of the present invention includes, after the above-described method for assisting in prognosis prediction, a prognosis evaluation step of evaluating the prognosis of the subject based on the ratios of neoplastic follicle regulatory T cells (Tnfr), CD4-positive neoplastic follicle cytotoxic T cells (Tnfc4), and CD8-positive neoplastic follicle cytotoxic T cells (Tnfc8) among the T cells collected from the subject.

In the prognosis evaluation step, if the ratios of neoplastic follicle regulatory T cells (Tnfr), CD4-positive neoplastic follicle cytotoxic T cells (Tnfc4), and CD8-positive neoplastic follicle cytotoxic T cells (Tnfc8) among the T cells collected from the subject are less than 2.0% for Tnfr and less than 2.5% for Tnfc (total of Tnfc4 and Tnfc8), the patient is evaluated as being at high risk (poor prognosis), and if Tnfr is 2.0% or more and Tnfc is 2.5% or more, the patient is evaluated as being at low risk (cannot be said to have a poor prognosis).

The prognosis evaluation step is a step carried out in vitro.

Alternatively, the method for predicting the prognosis of follicular lymphoma in a subject of the present invention includes:
a quantification step of quantifying neoplastic follicle regulatory T cells (Tnfr) and neoplastic follicle cytotoxic T cells (Tnfc) among T cells collected from a subject by the above-mentioned quantification method;
a ratio calculation step of calculating the ratios of neoplastic follicle regulatory T cells (Tnfr), CD4-positive neoplastic follicle cytotoxic T cells (Tnfc4), and CD8-positive neoplastic follicle cytotoxic T cells (Tnfc8) among the T cells collected from the subject based on the quantification results obtained in the quantification step; and
a prognosis evaluation step of evaluating the prognosis of the subject based on the ratios calculated in the ratio calculation step, wherein
in the prognosis evaluation step, if the ratios calculated in the ratio calculation step are less than 2.0% for the neoplastic follicle regulatory T cells and less than 2.5% for the sum of the CD4-positive neoplastic follicle cytotoxic T cells and the CD8-positive neoplastic follicle cytotoxic T cells, the prognosis is evaluated as poor, and if Tnfr is 2.0% or more and Tnfc is 2.5% or more, the patient is evaluated as having a low risk (the prognosis cannot be said to be poor).

Furthermore, by combining with existing indices such as the conventional FLIPI, FLIPI2 or the like, more accurate prognosis predictions can be made, and it is also possible to identify subjects (patients) with extremely good prognosis or extremely poor prognosis.

In one embodiment, the present invention may further include other steps after the prognosis evaluation step of the method for predicting prognosis of the present embodiment. Such other steps may include a step of treating a patient with follicular lymphoma based on the results of the prognosis prediction, or the like. When the method for predicting prognosis of the present embodiment further includes a treatment step in addition to the prognosis evaluation step, the method for predicting prognosis of the present embodiment may be a method for treating a patient with follicular lymphoma.

In the treatment step, the treatment policy for follicular lymphoma can be determined based on the evaluation results of the method for predicting prognosis of the present embodiment. Treatment methods for follicular lymphoma patients evaluated as having a poor prognosis include the same treatment methods as those for follicular lymphoma patients described above.

### (Examples)

The present invention will be described in more detail below with reference to Experimental Examples, but the present invention is not limited to the examples described below.

### [Experimental Example 1] Experiment by flow cytometry method

A method similar to the flow cytometry method, which is performed as a routine clinical test, was used.

Clinical lymphoma samples were processed to single cells and then subjected to flow cytometry using anti-CD3, anti-CD4, anti-CD8a, anti-PD-1, anti-CD25, and anti-TIM-3 antibodies.

CD4-positive cells and CD8-positive cells were detected and enumerated from the T cells collected from patients (FIG. 1).

Neoplastic follicle regulatory T cells (Tnfr) were detected as a CD4⁺ (CD3⁺) PD-1⁺ CD25⁺ population. Furthermore, CD8-positive neoplastic follicle cytotoxic T cells (Tnfc8) were detected as a CD8⁺ PD-1⁺ TIM-3⁻ population. The ratios of these cells in the total T cells (CD3-positive cells or CD4-positive cells, and CD8-positive cells) were calculated.

### [Experimental Example 2] Experiment using multiplex immunostaining method

A method similar to the multiplex immunostaining method, which is performed as a routine clinical test, was used.

Formalin-fixed, paraffin-embedded clinical lymphoma samples were subjected to multiplex immunostaining using anti-CD3e, anti-CD4, anti-CD8, anti-PD-1, anti-FOXP3, anti-TCF-1, and anti-Granzyme K antibodies with the PhenoCycler-Fusion system (manufactured by AKOYA).

The antibodies were prepared according to the protocol recommended by AKOYA, and the experiments were performed (FIG. 2: a. Multiplex immunostaining). Neoplastic follicle regulatory T cells (Tnfr) were detected as CD4⁺ PD-1⁺ FOXP3⁺ populations (FIG. 2: b. Tnfr detection). Furthermore, Neoplastic follicle cytotoxic T cells (Tnfc) were detected as CD8⁺ Granzyme K⁺ PD-1⁺ TCF-1⁺ or CD4⁺ Granzyme K⁺ PD-1⁺ TCF-1⁺ populations (FIG. 2: c. Tnfc detection).

### [Experimental Example 3] Specific examples of prognosis prediction

FIGs. 3A and 3B show specific examples of stratification of the time to recurrence in follicular lymphoma patients based on the ratios of neoplastic follicle regulatory T cells (Tnfr) and neoplastic follicle cytotoxic T cells (Tnfc) calculated by multiplex immunostaining using the PhenoCycler-Fusion system (manufactured by AKOYA).

The time to recurrence was significantly longer in patients with high ratios of both Tnfr and Tnfc cells, and particularly long in patients with high ratios of both types of cells, and particularly short in patients with low ratios of both types. This result was confirmed in three independent patient cohorts (FIG. 3A, "Original Cohort", "Validation Cohort 1", and FIG. 3B, "Validation Cohort 2"). This prognosis prediction was also independent of FLIPI, and its effectiveness was confirmed in both patient cohorts classified as low risk (FLIPI low-risk) and high risk (FLIPI high-risk) by FLIPI risk classification (FIG. 4).

### [Experimental Example 4]

(1) The expression of inhibitory factor genes in Tnfr and follicle regulatory T cells from normal lymph nodes were compared using single-cell RNA sequencing data.
   FIG. 5: a shows violin plots of inhibitory factor gene expression in Tnfr and follicle regulatory T cells from normal lymph nodes, demonstrating that the expression of inhibitory genes is elevated in Tnfr.
(2) The inhibitory effect of Tnfr cells on the activation and differentiation of Tfh cells was examined.
   Tfh cells promote the progression of follicular lymphoma by actively interacting with neoplastic B cells, which are cancer cells. When Tfh and Tnfr cells were cocultured under T cell receptor (TCR) stimulation, the ratio of CD25-positive activated Tfh cells decreased compared to Tfh cells cultured alone (FIG. 5: b. The inhibitory effect of Tfh on the activation of Tfh cells), and the division activity of Tfh cells detected by a cell tracking reagent decreased (FIG. 5: c. The inhibitory effect of Tfh on the differentiation of Tfh cells).
(3) The inhibitory effect of Tnfr on the activation of neoplastic B cells and the inhibitory effect of Tnfr on the cell survival of neoplastic B cells were examined.

Neoplastic B cells were cultured alone, cocultured with Tfh cells, and cocultured with Tfh cells and Tnfr cells. As a result, it was confirmed that the coculture of neoplastic B cells with Tfh cells showed a greater activation effect on neoplastic B cells (FIG. 5: d. The inhibitory effect of Tnfr on the activation of neoplastic B cells) and a cell survival-promoting effect on neoplastic B cells (FIG. 5: e. The inhibitory effect of Tnfr on the cell survival of neoplastic B cells) compared to the culture of neoplastic B cells alone. Furthermore, the coculture of neoplastic B cells with Tfh cells and Tnfr cells showed a decrease in the ratio of cells positive for the activation marker CD86 of neoplastic B cells, i.e., a weakened activation effect of Tfh cells on neoplastic B cells (FIG. 5: d), and an increase in the ratio of cells positive for the cell death marker Annexin V of neoplastic B cells, i.e., a weakened cell survival-promoting effect of Tfh cells on neoplastic B cells (FIG. 5: e).

### [Experimental Example 5]

(1) A comparative analysis was performed on the gene expression between Tnfc8 and effector CD8 T cells (effector CD8 cells) or between Tnfc8 and exhausted CD8 T cells (exhausted CD8 cells). Gene expression information between Tnfc8 and effector CD8 cells or between Tnfc8 and exhausted CD8 cells was extracted and analyzed comparatively. Genes with differential expression were plotted in a volcano plot (FIG. 6: a. Comparison of gene expression between Tnfc8 and effector CD8 cells; b. Comparison of gene expression between Tnfc8 and exhausted CD8 cells). Tnfc8 highly expressed CXCL13, CXCR5, and BCL6 genes, which promote migration to and localization within follicular structures, and also highly expressed TCF7, which maintains cellular stemness. Compared to exhausted CD8 cells, the expression of exhaustion markers such as LAG3 and HAVCR2 was reduced, further confirming their stemness.
(2) Cellular stemness was examined by cell division assay.
   A cell tracking reagent that traces cell division was used to stimulate the T cell receptor (TCR) to induce cell division, and found that Tnfc8 cells maintained a high level of cell division ability compared to exhausted CD8 cells (FIG. 6: c. Cell division ability).
(3) Cytokine production ability, which is an index of cytotoxicity, was measured.
   The cells were stimulated with PMA (phorbol myristate acetate)/ionomycin and evaluated for their ability to produce interferon gamma (FIG. 6: d) and TNF (tumor necrosis factor) alpha (FIG. 6: e). This demonstrated that these cells maintained a higher level of production ability than naive CD8 cells.
(4) The activation-inhibitory effect and cytocidal effect of Tnfc8 on neoplastic B cells were examined.

Neoplastic B cells, which are cancer cells in follicular lymphoma, were cultured alone, cocultured with Tnfc8, cocultured with effector CD8 cells, and cocultured with exhausted CD8 cells. It was shown that the coculture with Tnfc8 significantly inhibited the activation of neoplastic B cells, although not as significantly as effector CD8 cells (FIG. 6 : f. Inhibitory effect of Tnfc8 on the activation of neoplastic B cells), and promoted cell death (FIG. 6 : g. Cytocidal effect of Tnfc8 on neoplastic B cells).

### [Experimental Example 6]

(1) (a) Experiment on induction of Tnfr from regulatory T cells by cytokine IL-21.
   Regulatory T cells in human lymph nodes were cultured under TCR stimulation in the presence of transforming growth factor (TGF) β1, with or without the addition of IL-21. As a result, high expression of CXCR5, which is a characteristic of Tnfr, was induced in the presence of IL-21 (FIG. 7: a. Induction of Tnfr phenotype by IL-21). Furthermore, regulatory T cells in human lymph nodes were cultured under TCR stimulation in the presence or absence of TGFβ1 and IL-21. As a result, high expression of CXCR5 and BCL6, which are characteristics of Tnfr, was induced in the presence of IL-21 (FIG. 8: a. Induction of Tnfr phenotype by IL-21).
(2) Experiment on induction of Tnfc8 from naive CD8 cells by cytokine IL-21.

Naive CD8 cells from human lymph nodes were cultured under TCR stimulation in the presence of TGFβ1, with or without the addition of IL-21. As a result, high expression of CXCR5 (top) and CCR7 (bottom), which are characteristics of Tnfc, was induced in the presence of IL-21 (FIG. 7: b. Induction of Tnfc8 phenotype by IL-21). Furthermore, naive CD8 cells from human lymph nodes were cultured under TCR stimulation in the presence or absence of TGFβ1 and IL-21. As a result, high expression of Granzyme M and TCF1, which are characteristics of Tnfc, was induced in the presence of IL-21 (FIG. 8: b. Induction of Tnfc8 phenotype by IL-21).

These cell induction methods are expected to improve the therapeutic efficacy by modifying current T-cell genetic engineering therapies and incorporating the specific characteristics of Tnfr and Tnfc cells.

While preferred embodiments of the present invention have been described and illustrated, it should be understood that these are exemplary of the present invention and should not be considered limiting. Additions, omissions, substitutions, and other modifications can be made without departing from the scope of the present invention. Accordingly, the present invention is not to be deemed limited by the foregoing description, but is limited only by the appended claims.

### INDUSTRIAL APPLICABILITY

By implementing the present invention, it becomes possible to more accurately predict the prognosis of follicular lymphoma patients at the time of their first visit.

It is also expected that neoplastic follicle regulatory T cells (Tnfr) and neoplastic follicle cytotoxic T cells (Tnfc) can be extracted and used for the development of therapeutics for use as direct treatments.

## Claims

1. A method for quantifying neoplastic follicle regulatory T cells and neoplastic follicle cytotoxic T cells among T cells collected from a subject for predicting prognosis of follicular lymphoma, comprising:
a flow cytometry step of detecting and enumerating neoplastic follicle regulatory T cells and neoplastic follicle cytotoxic T cells among T cells collected from a subject by a flow cytometry method using an anti-CD4 antibody, an anti-CD8a antibody, an anti-PD-1 antibody, an anti-CD25 antibody, and an anti-TIM-3 antibody; or
a multiplex immunostaining step of detecting and enumerating neoplastic follicle regulatory T cells and neoplastic follicle cytotoxic T cells among T cells collected from a subject by a multiplex immunoassay using an anti-CD4 antibody, an anti-CD8 antibody, an anti-PD-1 antibody, an anti-FOXP3 antibody, an anti-TCF-1 antibody, and an anti-Granzyme K antibody.

2. The method according to claim 1, wherein in the flow cytometry step, CD4⁺ PD-1⁺ CD25⁺ cells are detected and enumerated as neoplastic follicle regulatory T cells, and CD8a⁺ PD-1⁺ TIM-3⁻ cells are detected and enumerated as CD8-positive neoplastic follicle cytotoxic T cells.

3. The method according to claim 1, wherein in the multiplex immunostaining step, CD4⁺ PD-1⁺ FOXP3⁺ cells are detected and enumerated as neoplastic follicle regulatory T cells, CD8⁺ Granzyme K⁺ PD-1⁺ TCF-1⁺ cells are detected and enumerated as CD8-positive neoplastic follicle cytotoxic T cells, and CD4⁺ Granzyme K⁺ PD-1⁺ TCF-1⁺ cells are detected and enumerated as CD4-positive neoplastic follicle cytotoxic T cells.

4. A method for screening neoplastic follicle regulatory T cells and neoplastic follicle cytotoxic T cells from T cells collected from a subject for predicting prognosis of follicular lymphoma, comprising:
a flow cytometry step of detecting and enumerating neoplastic follicle regulatory T cells and neoplastic follicle cytotoxic T cells among T cells collected from a subject by a flow cytometry method using an anti-CD4 antibody, an anti-CD8a antibody, an anti-PD-1 antibody, an anti-CD25 antibody, and an anti-TIM-3 antibody; or
a multiplex immunostaining step of detecting and enumerating neoplastic follicle regulatory T cells and neoplastic follicle cytotoxic T cells among T cells collected from a subject by a multiplex immunoassay using an anti-CD4 antibody, an anti-CD8 antibody, an anti-PD-1 antibody, an anti-FOXP3 antibody, an anti-TCF-1 antibody, and an anti-Granzyme K antibody.

5. The method according to claim 4, wherein in the flow cytometry step, CD4⁺ PD-1⁺ CD25⁺ cells are detected and enumerated as neoplastic follicle regulatory T cells, and CD8a⁺ PD-1⁺ TIM-3⁻ cells are detected and enumerated as CD8-positive neoplastic follicle cytotoxic T cells.

6. The method according to claim 4, wherein in the multiplex immunostaining step, CD4⁺ PD-1⁺ FOXP3⁺ cells are detected and enumerated as neoplastic follicle regulatory T cells, CD8⁺ Granzyme K⁺ PD-1⁺ TCF-1⁺ cells are detected and enumerated as CD8-positive neoplastic follicle cytotoxic T cells, and CD4⁺ Granzyme K⁺ PD-1⁺ TCF-1⁺ cells are detected and enumerated as CD4-positive neoplastic follicle cytotoxic T cells.

7. A therapeutic composition for treating follicular lymphoma, comprising T cells detected as CD4⁺ PD-1⁺ CD25⁺ cells or CD8a⁺ PD-1⁺ TIM-3⁻ cells in a flow cytometry step of detecting and enumerating neoplastic follicle regulatory T cells and neoplastic follicle cytotoxic T cells among T cells collected from a subject by a flow cytometry method using an anti-CD4 antibody, an anti-CD8a antibody, an anti-PD-1 antibody, an anti-CD25 antibody, and an anti-TIM-3 antibody.

8. A therapeutic composition for treating follicular lymphoma, comprising T cells detected as CD4⁺ PD-1⁺ FOXP3⁺ cells, CD8⁺ Granzyme K⁺ PD-1⁺ TCF-1⁺ cells, or CD4⁺ Granzyme K⁺ PD-1⁺ TCF-1⁺ cells in a multiplex immunostaining step of detecting and enumerating neoplastic follicle regulatory T cells and neoplastic follicle cytotoxic T cells among T cells collected from a subject by a multiplex immunoassay using an anti-CD4 antibody, an anti-CD8 antibody, an anti-PD-1 antibody, an anti-FOXP3 antibody, an anti-TCF-1 antibody, and an anti-Granzyme K antibody.

9. The therapeutic composition according to claim 7 or 8, wherein the T cells are T cells pre-cultured with IL-21.

10. The therapeutic composition according to claim 7 or 8, wherein the T cells are artificially genetically modified T cells.

11. A kit for predicting prognosis of follicular lymphoma, comprising:
an antibody set comprising an anti-CD4 antibody, an anti-CD8a antibody, an anti-PD-1 antibody, an anti-CD25 antibody, and an anti-TIM-3 antibody; or
an antibody set comprising an anti-CD4 antibody, an anti-CD8 antibody, an anti-PD-1 antibody, an anti-FOXP3 antibody, an anti-TCF-1 antibody, and an anti-Granzyme K antibody.

12. The kit according to claim 11, which is for identifying, detecting, or screening neoplastic follicle regulatory T cells and neoplastic follicle cytotoxic T cells from a population of T cells collected from a subject.

13. A method for assisting in predicting prognosis of follicular lymphoma in a subject, comprising:
a flow cytometry step of detecting and enumerating neoplastic follicle regulatory T cells and neoplastic follicle cytotoxic T cells among T cells collected from a subject by a flow cytometry method using an anti-CD4 antibody, an anti-CD8a antibody, an anti-PD-1 antibody, an anti-CD25 antibody, and an anti-TIM-3 antibody; and
a ratio calculation step of calculating ratios of neoplastic follicle regulatory T cells, CD4-positive neoplastic follicle cytotoxic T cells, and CD8-positive neoplastic follicle cytotoxic T cells among the T cells collected from the subject.

14. The method according to claim 13, wherein in the ratio calculation step, the ratio of CD8-positive neoplastic follicle cytotoxic T cells is calculated based on results of detecting and enumerating CD4-positive neoplastic follicle cytotoxic T cells.

15. A method for assisting in prognosis prediction of follicular lymphoma in a subject, comprising:
a multiplex immunostaining step of detecting and enumerating neoplastic follicle regulatory T cells and neoplastic follicle cytotoxic T cells among T cells collected from a subject by a multiplex immunoassay using an anti-CD4 antibody, an anti-CD8 antibody, an anti-PD-1 antibody, an anti-FOXP3 antibody, an anti-TCF-1 antibody, and an anti-Granzyme K antibody; and
a ratio calculation step of calculating ratios of neoplastic follicle regulatory T cells, CD4-positive neoplastic follicle cytotoxic T cells, and CD8-positive neoplastic follicle cytotoxic T cells among the T cells collected from the subject.

16. A method for predicting prognosis of follicular lymphoma in a subject, comprising:
a quantifying step of quantifying neoplastic follicle regulatory T cells and neoplastic follicle cytotoxic T cells among T cells collected from a subject by the method according to any one of claims 1 to 6;
a ratio calculation step of calculating ratios of neoplastic follicle regulatory T cells, CD4-positive neoplastic follicle cytotoxic T cells, and CD8-positive neoplastic follicle cytotoxic T cells among the T cells collected from the subject based on quantification results obtained in the quantification step; and
a prognosis evaluation step of evaluating the prognosis of the subject based on the ratios calculated in the ratio calculation step, wherein
in the prognosis evaluation step, if the ratios calculated in the ratio calculation step are less than 2.0% for neoplastic follicle regulatory T cells and less than 2.5% for a sum of the CD4-positive neoplastic follicle cytotoxic T cells and the CD8-positive neoplastic follicle cytotoxic T cells, the prognosis is assessed as poor.
